# EUROPEAN PATENT SPECIFICATION

(11) **EP 1 581 199 B1**
(45) Date of publication and mention of the grant of the patent: **25.02.2015**
(21) Application number: 03785600.2
(22) Date of filing: 01.09.2003
(51) Int. Cl.: A61K 31/00, A23K 1/16, A23L 1/30, A61K 31/375, A61K 31/355, A23L 1/302, A23L 1/304, A23L 1/305, A23K 1/18, A23L 1/09, A23L 1/29, A61K 36/82, A61K 36/87, A61K 31/198, A61K 31/205, A61K 31/7076, A61K 36/16, A61K 36/258, A61K 36/48, A61K 36/74, A61K 31/19

(54) **ORALLY ADMINISTRABLE COMPOSITION FOR IMPROVING SKIN QUALITY**
ORAL VERABREICHBARE ZUSAMMENSETZUNG ZUR VERBESSERUNG DER HAUTQUALITÄT
COMPOSITION PAR VOIE ORALE D'AMELIORATION DE LA QUALITE DE LA PEAU

(30) Priority: 09.09.2002 EP 02078707
(43) Date of publication of application: 05.10.2005
(73) Proprietor: Nestec S.A., 1800 Vevey (CH)
(72) Inventor: SMOLA, Hans, D- 50678 Koeln (DE); PRIDMORE-MERTEN, Sylvie, CH-1005 Lausanne (CH); LURATI, Emmanuelle, CH-1260 Nyon (CH)
(74) Representative: Rupp, Christian
(86) International application number: PCT/EP2003/009687
(87) International publication number: WO 2004/026287

(56) References cited:
- EP-A- 0 845 266
- WO-A-02/11745
- WO-A-98/00024
- FR-A- 2 818 136
- US-A- 5 656 588
- US-A1- 2001 043 983
- FREI V ET AL: "ACTIVATION OF FIBROBLAST METABOLISM IN A DERMAL AND SKIN EQUIVALENT MODEL: A SCREENING TEST FOR ACTIVITY OF PEPTIDES" INTERNATIONAL JOURNAL OF COSMETIC SCIENCE, BLACKWELL SCIENTIFIC PUBLICATIONS, OXFORD, GB, vol. 20, no. 3, June 1998 (1998-06), pages 159-173, XP001128165 ISSN: 0142-5463

## Description

The present invention pertains to a method for improving skin turgor or for preventing or restoring skin age-related alterations in humans or animals.

### Background of the Invention

During the lifetime of a living being different signs, characteristic of aging, appear on the skin, with the principal clinical signs being the appearance of fine lines and deep wrinkles which increase or are accentuated with age. Moreover, the skin's complexion is generally modified and diffuse irritations and occasionally telangiectasias may come into existence on certain areas. These signs of ageing are even promoted by exposure of the skin to exogenous influences, such as e.g. UV-radiation, pollutants, free radicals or chemical substances.

Skin aging is reflected by major structural changes and variations in composition. Most notably aged skin has less collagen and glycosaminoglycans compared with young skin (Fenske NA, Lober CW: J Am Acad Dermatol 15:571-85 1986).

On the molecular level the connective tissue is predominantly composed of collagens, proteoglycans and hyaluronic acid. While the collagens form a basket like three-dimensional mesh, the proteoglycans are embedded within. Proteoglycans are composed of a core protein to which numerous glycosaminoglycan side chains, repeating non-branched disaccharides, are covalently attached. One component of the disaccharide unit is an aminosugar hence the name GAG. The amount of carbohydrate in a proteoglycan can comprise up to 95% of its weight. Concomitant is the huge water binding capacity of proteoglycans. In skin these molecules compartimentalize interstitial fluid water in the dermis. Their swelling properties within the collagen meshwork build up an internal pressure which smoothes the skin surface and generates the skin turgor.

Several attempts have been made to inhibit the aging process or even revert the occurred alterations. Topical application of pharmacological substances or cosmetic ingredients to date has produced best results. In this respect FR 2808682 provides a novel cosmetic product especially for skin care, which contains fresh, polyphenol-containing vine cells as active agent, together with a carrier suitable for topical application.

Also, DE 10108097 provides a cosmetic formulation, especially for use on aging and/or stressed skin, which contains water and substance(s) forming lamellar structures with water, also contains compound(s) (I) with trimethylammoniummethyl group(s) of formula (IA), metabolites of (I) and/or S-adenosylmethionine.

US 2002/0041890 discloses cosmetic skin care methods and compositions containing phosphates and/or sulfates of branched alcohols and/or ethoxylates thereof. These compositions provide control of sebum secretion from sebocytes, improved oil control and improved skin feel, prevent shine and stickiness, while also providing anti-aging benefits which results in reduced appearance of wrinkles and aged skin, improved skin colour, treatment of photoaged skin, improvement in skin's radiance and clarity and finish, and an overall healthy and youthful appearance of the skin.

EP 0 845 266 discloses an anti-oxidative composition with sweet whey concentrate.

US 5,656,588 describes a composition comprising a source of carnosine for stimulating and improving wound healing.

FR 2 818 136 discloses a cosmetic composition comprising 7-hydroxy DHEA or 7-keto DHEA and at least one inhibitor of NO-synthase.

US 2001/0043983 describes a composition having anti-aging effects and comprising R-alpha-lipoic acid and L-carnitine.

WO 02/11745 relates to a composition comprising ginkgo biloba extract that inhibits angiogenesis and matrix metalloproteinase.

WO 98/00024 describes nutritional supplements for increasing the levels of high density lipoprotein and calcium ions comprising antioxidants, a specific green barley composition, and a particular tincture of ginkgo biloba extract.

Murad et al. describe the effect of an oral supplement containing glucosamine, amino acids, minerals and antioxidants on cutaneous aging (Journal of Dermatological Treatment, 2001, p. 47-51).

US 2002/0048798 describe novel antioxidants.

Another means to prevent skin deterioration or ageing, respectively, is to provide compounds scavenging free radicals. In this respect EP 0 761 214 discloses singlet oxygen quenchers comprising aniline derivatives and difurfuryl amine derivatives, which are reported to reduce the oxidative stress to the skin.

Some medicaments have also been developed in this regard. For example, EP 1230952 provides a method of preparing a medicament comprising an estrogen and a progestogen for use in delaying the onset and treating skin aging.

On the other hand, few dietary compositions have already been described. In US 6365175, edible compositions containing petroselinic acid are used for the preparation of food compositions or food supplements that are used as antiinflammatory compositions that inhibit the production of metabolites of arachidonic acid and/or reduces the formation of intracellular adhesion molecules or as anti-aging compositions with a positive impact on wrinkling, sagging, photodamaged skin, dry skin, flaky skin and age spots.

There is still a need in the art to provide an effective nutritional way for improving skin quality and preventing or reverting alterations due to aging process.

Accordingly, an object of the present invention is to provide such means in order to improve skin quality.

This problem has been solved by providing orally administrable compositions, that are capable to stimulate extracellular matrix production, particularly synthesis of components such as glycosaminoglycans that bind interstitial fluid and thus improve skin turgor.

### Summary of the Invention

The invention relates to a non-therapeutic, cosmetic method to improve skin turgor in humans or to prevent or restore skin age-related alterations in humans comprising administering to the individual a nutritionally complete human food or a dietary supplement which comprises as an active ingredient an effective amount of a molecule that stimulates energy metabolism of the cell selected from L-carnitine, creatine, cardiolipin, or nicotinamide and natural sources thereof in an orally acceptable carrier.

The invention also relates to a non-therapeutic, cosmetic method to improve skin turgor in animals or to prevent or restore skin age-related alterations in animals comprising administering to the individual a nutritionally complete animal food or a dietary supplement which comprises as an active ingredient an effective amount of a molecule that stimulates energy metabolism of the cell selected from L-carnitine, creatine, cardiolipin, or nicotinamide and natural sources thereof, in an orally acceptable carrier.

The composition of the method can further comprise fatty acids (monounsaturated and polyunsaturated, particularly omega-3 fatty acids) or carbohydrate.

The composition of the method can further comprise an antioxidant selected from lipoic acid, cysteine, cystine, methionine, S-adenosyl-methionine, taurine, glutathione or natural sources thereof, or carotenoids, ubiquinones, tea catechins, coffee extracts containing polyphenols and/or diterpenes, grape seed extracts rich in proanthocyanidins, spice extracts, soy extracts or ursodeoxycholic acid, ursolic acid, ginseng and gingenosides and natural sources thereof.

The composition of the method can comprise carnitine.

The composition of the method can comprise gingko extract.

### Detailed Description

Disclosed is an orally administrable composition for improving skin quality, which comprises as an active ingredient an effective amount of a molecule that stimulates energy metabolism of the cell, an antioxidant or combinatory admixtures thereof, in an orally acceptable carrier, is concerned.

The molecule that stimulates energy metabolism of the cell may be L-carnitine, creatine, fatty acids (mono or polyunsaturated fatty acids, particularly omega-3 fatty acids), cardiolipin, nicotinamide, carbohydrate and natural sources thereof, for example.

The amount of said molecule can be of at least 1mg per kg of body weight per day, more preferably from 1mg to 1 g per kg of body weight per day.

The antioxidants are compounds that decrease protein oxidation (e.g. prevent formation of protein carbonyls). They may be sources of thiols (e.g. Lipoic acid, cysteine, cystine, methionine, S-adenosyl-methionine, taurine, glutathione and natural sources thereof), or compounds that upregulate their biosynthesis in vivo, for example. The antioxidant may also be other antioxidants such as vitamin C, vitamin E (tocopherols and tocotrienols), carotenoids (carotenes, lycopene, lutein, zeaxanthine..) ubiquinones (e.g.CoQ10), tea catechins (e.g epigallocatechin gallate), coffee extracts containing polyphenols and/or diterpenes (e.g. kawheol and cafestol), ginkgo biloba extracts, grape or grape seed extracts rich in proanthocyanidins, spice extracts (e.g. rosemary), soy extracts containing isoflavones and related phytoestrogens and other sources of flavonoids with antioxidant activity, compounds that upregulate cell antioxidant defense (e.g. ursodeoxycholic acid for increased glutathione S-transferase, ursolic acid for increased catalase, ginseng and gingenosides for increase superoxide dismutase and natural sources thereof i.e. herbal medicines).

The amount of the antioxidant can be of at least 0.025 mg per kg of body weight per day, more preferably from 0.025 mg to 250mg per kg of body weight per day.

The carrier may be any food or pharmaceutical product, or a nutritional supplement or a composition for oral administration. Examples for food or pharmaceuticals carriers are milk, yoghurt, curd, cheese, fermented milks, milk based fermented products, ice-creams, fermented cereal based products, milk based powders, infant formulae or tablets, liquid suspensions, dried oral supplement, wet oral supplement, dry-tube-feeding, pet food products. The composition for oral administration may be in capsules, soft capsules, tablets, pastes or pastilles, gums, or drinkable solutions or emulsions. Methods for preparing the carrier are common knowledge.

The composition may also comprise usual excipients, in particular sweeteners, flavouring agents or preservatives. It can further comprise a prebiotic and/or a probiotic microorganism.

The compositions may be formulated according to any technique that is well known to this art.

Disclosed is that a pharmaceutical composition containing at least one of the active components in an amount sufficient to achieve the desired effect in an individual can be prepared. This composition may be a tablet, a liquid, a dried oral supplement, a wet oral supplement, dry tube-feeding, wet tube-feeding etc.. The pharmaceutical composition will further contain carriers and excipients that are suitable for delivering the respective active molecule of different nature to the target tissue. The kind of the carner/excipient and the amount thereof will depend on the nature of the substance and the mode of drug delivery and/or administration contemplated. It will be appreciated that the skilled person will, based on his own knowledge select the appropriate components and galenic form to target the active compound to the skin.

Disclosed is that a food composition for human consumption is prepared. This composition may be a nutritional complete formula, a dairy product, a chilled or shelf stable beverage, soup, a dietary supplement, a meal replacement, and a nutritional bar or a confectionery.

The nutritional formula can be enterally administrable; for example in the form of a powder, a liquid concentrate, or a ready-to-drink beverage. If it is desired to produce a powdered nutritional formula, the homogenised mixture is transferred to a suitable drying apparatus such as a spray drier or freeze drier and converted to powder.

A usual food product may be enriched with the combination. For example, a fermented milk, a yoghurt, a fresh cheese, a renneted milk, a confectionery bar, breakfast cereal flakes or bars, drinks, milk powders, soy-based products, non-milk fermented products or nutritional supplements for clinical nutrition. Then, the amount of the molecule that stimulates energy metabolism is preferably of at least 50 ppm by weight and can be up to 2000 mg per day and and the antioxidant is preferably of at least 10 ppm by weight.

A pet food products may be prepared. The petfood formulation is preferably a complete and nutritionally balanced pet food. It can also be a dietary supplement for pets or in the form of a pharmaceutical composition. The nutritionally complete pet food formulation may be in any suitable form, for example a powder, a dried kibble, or pellet or other dried form, extruded form, semi-moist or wet form, such as a chunk or loaf or pudding. It may be chilled or provided as a shelf stable product. This pet food may be produced by conventional methods.

Dietary supplements may be prepared so as to improve pet food quality. As dietary adjuncts, they may be encapsulated or may be provided in powder form and packaged in conjunction with or separately from a main meal, be it wet or dry. By way of example, a powder containing selected substances, may be packed in sachets in a powder form or in a gel or lipid or other suitable carrier. These separately packaged units may be provided together with a main meal or in multi-unit packs for use with a main meal or treat, according to user instructions.

The food composition aims to improve skin hydratation, skin elasticity, skin appearance, and reduce or revert skin dryness, wrinkling, pore size and skin roughness.

Disclosed is the use of a (list of compounds used will be added if OK), for the preparation of an orally administrable composition intended to improve skin quality and prevent or restore age-related alterations in humans or in animals.

Disclosed is a method to improve skin quality and prevent or restore age-related alterations of skin in humans or animals, comprising administering to the individual, a composition as described above.

The said composition may be administered to the mammal as a supplement to the normal diet or as a component of a nutritionally complete food. It is disclosed to prepare a nutritionally complete food as described above.

The amount of the composition to be consumed by the individual to obtain a beneficial effect will depend upon its size, its type, and its age. However an amount of carnitine of at least 1mg per kg of body weight per day and an amount of the antioxidant of at least 0.025 mg per kg of body weight per day, would usually be adequate.

The following examples are given by way of illustration only and in no way should be construed as limiting the subject matter of the present application. All percentages are given by weight unless otherwise indicated.

### Example 1: In-vivo trials on the effect of dietary nutrients according to the present invention

### • Study design:

Dietary intervention was of 3 months, all animal groups were fed Ad lib. Animal weight was measured once a week.

### • Animals:

Male mice C57/BL6 were obtained from Iffa credo (France) at 9 weeks of age. After 3 weeks adaptation, mice (12 weeks old) were housed individually and fed the control diet A until the nutritional intervention. At 12 months of age the mice were randomised in 5 groups (A, C, D and E) of 10 mice. Dietary intervention was of 3 months; mice had free access to food and water and were submitted to 12 hours light and dark cycles.

### • Diets:

The control diet (diet A) composed of 18% proteins (soy and whey), 11% fat (soybean oil), 59% carbohydrates (starch + sucrose) and 10% cellulose was supplemented with either ginkgo biloba extract (diet D), or a cocktail of antioxidants comprising vitamin C, vitamin E, grape seed extract and cysteine (diet B) and/or L-carnitine (diet C and E respectively). These diets are as follows:
**Diet A - Control :** 18% proteins (soy and whey), 11% fat, 59% carbohydrates, 5% cellulose.
**Diet C - Cocktail of antioxidants :** Diet A + 0.19% vit C, 0.03% vit E, 0.075% grape seed extract, 0.4% cysteine.
**Diet D :** Diet A + 0.3% L- carnitine + cocktail of antioxidants of diet C.
**Diet E :** Diet A + 0.0375% Ginkgo biloba extract (Linnea)

### • Glycosaminoglycan determination:

After three month of dietary intervention the animals were sacrificed and standardised biopsies taken. Dorsal skin (midline, lower back) was collected freed from coat hair weighed, minced to 0.5 x 0.5 mm squares. These were extracted in 10 times volume of their weight for 24 hours in 0.1 % acetic acid, 1 M NaCl at 4oC with constant agitation. The samples were clarified from insoluble matter (remaining coat hair, epidermis) by centrifugation yielding an upper lipid layer, aqueous layer containing dissolved dermis and a pellet with insoluble matter. The aqueous layer was saved and protein determined. 25 microliter of this supernatant were supplemented with 200 microliter BCA reagent (BCA reagent A premixed with BCA reagent B according to the manufacturer's instructions, Pierce, Rockford, IL, USA) and incubated for 30 seconds with constant agitation. Colour development was measured at 562 nm protein concentration calculated from a BSA standard curve.

Glycosaminoglycans were determined in the extract according to the protocol of Chandrasekhar et al., Anal. Biochem. 161:103-108 1987). 50 microliter of the extract were mixed with 200 microliter of DMB reagent (19 mg dimethylene blue, 2.0 ml formic acid, 2.0 g Na-formate in 1000 ml double distilled water). Immediately the plates were read at 550 nm and 610 nm and the ratio OD 550/610 was measured. Dilutions of purified chondroitinsulfate were used as standard.

### Results

After dietary intervention for 3 months starting at the age of 12 month, the four experimental diets were compared to the control diet. Skin was extracted with an acidic, high salt buffer and GAGs as well as total protein determined in the extracts. GAG content in the extracts are presented in Table 1. Table 1 shows that glycosaminoglycan content in skin is stimulated in diets B, C, D and E over control diet A.

**Table 1. Glycosaminoglycan concentration in skin extracts (microg/ml)**

| **Glycosaminoglycan concentration (microg/ml)** | | |
|---|---|---|
| **Diets** | **Average (microg/ml)** | **Standard deviation (microg/ml)** |
| **A - control** | 1.07 | ± 0.05 |
| **C - antioxidant cocktail** | **1.34** | ± 0.04 |
| **D - carnitine + antioxidant cocktail** | **1.46** | ± 0.03 |
| **E - Gingko extract** | **1.59** | ± 0.09 |
| **F - carnitine** | **1.42** | ± 0.09 |

From these data the GAG values per gram of wet skin were derived and are displayed in Table 2.

**Table 2. Glycosaminoglycan concentration in skin (microg/g wet skin)**

| **Diets** | | **A** | **B** | **C** | **D** | **E** |
|---|---|---|---|---|---|---|
| **GAG (microg/g of skin)** | | | | | | |
| **15 months** | **average** | **10.7** | **13.4** | **14.6** | **15.9** | **14.2** |
| | SD | 0.53 | 0.44 | 0.31 | 0.92 | 0.99 |
| | median | 10.9 | 13.3 | 14.6 | 16.0 | 14.4 |

Table 2 shows that GAG content was increased by more than 50% in diet D and more than 40% in diets C and F compared to the control diet A.

Total protein extracted was also determined after the three 3 month dietary intervention in starting in animals of 12 month of age. The results calculated per gram of wet skin are given in Table 3.

**Table 3. total protein concentration in skin extracts (microg/g wet skin)**

| **Diets** | | **A** | **B** | **C** | **D** | **E** |
|---|---|---|---|---|---|---|
| **Total protein microg/g of skin** | | | | | | |
| **15 months** | **average** | **6353** | **6240** | **4451** | **6906** | **5999** |
| | SD | 944 | 1061 | 340 | 2573 | 1359 |
| | median | 6190 | 5671 | 4513 | 6360 | 6021 |

Protein extracted varied less between diets compared with GAG content. Diet C had an almost 30% lower protein content compared with the control diet A. The changes of the different diets and control diet A were much less pronounced not exceeding 10%.

Compared with 6-month old mice receiving control diet A and the control diet A group in the 15-month old group, the results of the GAG and protein concentration are calculated as shown in Table 4.

**Table 4: Comparison of GAG and total protein content in skin extracts of 6-month and 15-month old animals**

| | **Diet A** | **Diet B** | **Diet C** | **Diet D** |
|---|---|---|---|---|
| | **6 months** | **15 months** | **6 months** | **15 months** |
| | **GAG microg/g of skin** | | **Total protein microg/g of skin** | |
| **Average** | **18.6** | **10.7** | **8637** | **6353** |
| SD | 1.10 | 0.53 | 2296 | 944 |
| median | 18.9 | 10.9 | 8321 | 6190 |

There is an age dependent decrease of GAG as well as extracted protein concentration in the 15 month old mice. The GAG concentration decrease is much more pronounced that that for the extracted protein content.

### Conclusion

From these data dietary intervention for three month in 12 month old mice with different diets B, C, D and E is able to reverse the age-dependent decrease in GAG content. The dietary intervention was efficient to increase the GAG content towards a pattern observed in young skin. As GAGs are potent molecules to bind large quantities of interstitial fluid they can increase the interstitial hydration and revert signs of skin ageing.

### Example 2: Dry pet food

A feed mixture is made up of about 58% by weight of corn, about 5.5% by weight of corn gluten, about 22% by weight of chicken meal, 2,5% dried chicory, 1% carnitine, 0.1% Vit C, vit E (150 IU / kg), 0.05%grape seed proanthocyanidin extract and 1% cysteine as antioxidant, salts, vitamins and minerals making up the remainder.

The fed mixture is fed into a preconditioner and moistened. The moistened feed is then fed into an extruder-cooker and gelatinised. The gelatinised matrix leaving the extruder is forced through a die and extruded. The extrudate is cut into pieces suitable for feeding to dogs, dried at about 110°C for about 20 minutes, and cooled to form pellets.

This dry dog food is able to improve the skin quality in dogs.

### Example 3: Dry pet food

A feed mixture is prepared as in example 2, using 2% carnitine and 0.05% ginkgo biloba extract as antioxidant. Then, the fed mixture is processed as in example 2. The dry dog food is particularly intended to improve or restore the age-related skin alterations in dogs.

### Example 4: Nutritional formula

A nutritional composition is prepared, and which contains for 100 g of powder: 15 % of protein hydrolysate, 25 % of fats, 55 % carbohydrates (including maltodextrin 37 %, starch 6 %, sucrose 12 %), traces of vitamins and oligoelements to meet daily requirements, 2 % minerals and 3 % moisture and 2% pyruvate and 1% carnosine or carnosine precursor as antioxidant.

13 g of this powder is mixed in 100 ml of water. The obtained formula is particularly intended for improving skin quality and preventing skin age-related alterations in humans.

### Example 5: Oral supplement

A daily orally administrable composition for improving skin quality, in particular that stimulates glycosamonoglycan production and deposition in skin contains 240 mg of Gingko biloba extract and Glucidex IT 19 (maltodextrin powder) QSP 500 mg.

The composition provides a protective and preventive effect on the alterations of the skin, in particular due to aging process.

## Claims

1. A non-therapeutic, cosmetic method to improve skin turgor in humans or to prevent or restore skin age-related alterations in humans comprising administering to the individual a nutritionally complete human food or a dietary supplement which comprises as an active ingredient an effective amount of a molecule that stimulates energy metabolism of the cell selected from L-carnitine, creatine, cardiolipin, or nicotinamide and natural sources thereof in an orally acceptable carrier.

2. A non-therapeutic, cosmetic method to improve skin turgor in animals or to prevent or restore skin age-related alterations in animals comprising administering to the individual a nutritionally complete animal food or a dietary supplement which comprises as an active ingredient an effective amount of a molecule that stimulates energy metabolism of the cell selected from L-carnitine, creatine, cardiolipin, or nicotinamide and natural sources thereof, in an orally acceptable carrier.

3. The method of claims 1 or 2, said composition further comprising fatty acids (monounsaturated and polyunsaturated, particularly omega-3 fatty acids) or carbohydrate.

4. The method of claims 1 or 2, said composition further comprising an antioxidant selected from lipoic acid, cysteine, cystine, methionine, S-adenosyl-methionine, taurine, glutathione or natural sources thereof, or carotenoids, ubiquinones, tea catechins, coffee extracts containing polyphenols and/or diterpenes, grape seed extracts rich in proanthocyanidins, spice extracts, soy extracts or ursodeoxycholic acid, ursolic acid, ginseng and gingenosides and natural sources thereof.

5. The method according to one of claims 1 to 4, which comprises carnitine.

6. The method according to one of claims 1-5, which comprises gingko extract.

## Patentansprüche

1. Nicht therapeutisches, kosmetisches Verfahren zur Verbesserung des Hautturgors bei Menschen oder zur Vorbeugung von oder Wiederherstellung bei altersbedingten Veränderungen der Haut beim Menschen, aufweisend die Verabreichung einer vollwertigen Nahrung oder einer Nahrungsergänzung an die Person, die als Wirkstoff eine wirksame Menge eines Moleküls aufweist, die den Energiemetabolismus der Zelle stimuliert, ausgewählt aus L-Carnitin, Creatin, Cardiolipin oder Nicotinamid und deren natürlichen Quellen, in einem oral verträglichen Träger.

2. Nicht therapeutisches, kosmetisches Verfahren zur Verbesserung des Hautturgors bei Tieren oder zur Vorbeugung von oder Wiederherstellung bei altersbedingten Veränderungen der Haut bei Tieren, aufweisend die Verabreichung einer vollwertigen Tiemahrung oder einer Nahrungsergänzung an das Tier, die als Wirkstoff eine wirksame Menge eines Moleküls aufweist, die den Energiemetabolismus der Zelle stimuliert, ausgewählt aus L-Carnitin, Creatin, Cardiolipin oder Nicotinamid und deren natürlichen Quellen, in einem oral verträglichen Träger.

3. Verfahren nach Anspruch 1 oder 2, wobei die Zusammensetzung weiterhin Fettsäuren (einfach und mehrfach ungesättigt, insbesondere Omega-3-Fettsäuren) oder Kohlenhydrate aufweist.

4. Verfahren nach Anspruch 1 oder 2, wobei die Zusammensetzung weiterhin ein Antioxidans aufweist, das aus folgenden ausgewählt ist: Liponsäure, Cystein, Cystin, Methionin, S-Adenosyl-Methionin, Taurin, Glutathion oder deren natürliche Quellen, oder Carotinoide, Ubichinone, Teekatechine, Polyphenole und/oder Diterpene enthaltende Kaffeeextrakte, Traubenkernextrakte, reich an Proanthocyanidinen, Gewürzextrakte, Sojaextrakte oder Ursodesoxycholsäure, Ursolsäure, Ginseng und Ginsenoside und deren natürliche Quellen.

5. Verfahren nach einem der Ansprüche 1 bis 4, das Carnitin aufweist.

6. Verfahren nach einem der Ansprüche 1-5, das Gingko-Extrakt aufweist.

## Revendications

1. Procédé cosmétique non-thérapeutique pour améliorer la turgescence cutanée chez les humains ou pour empêcher ou rétablir des altérations de la peau liées à l'âge chez les humains, comprenant l'administration à l'individu d'une nourriture pour humain nutritionnellement complète ou d'un complément alimentaire qui comprend comme ingrédient actif une quantité efficace d'une molécule qui stimule le métabolisme énergétique de la cellule choisie parmi la L-carnitine, la créatine, la cardiolipine, ou le nicotinamide et des sources naturelles de ceux-ci, dans un support convenant à la voie orale.

2. Procédé cosmétique non-thérapeutique pour améliorer la turgescence cutanée chez les animaux ou pour empêcher ou rétablir des altérations de la peau liées à l'âge chez les animaux, comprenant l'administration à l'individu d'une nourriture pour animal nutritionnellement complète ou d'un complément alimentaire qui comprend comme ingrédient actif une quantité efficace d'une molécule qui stimule le métabolisme énergétique de la cellule choisie parmi la L-carnitine, la créatine, la cardiolipine, ou le nicotinamide et des sources naturelles de ceux-ci, dans un support convenant à la voie orale.

3. Procédé selon les revendications 1 ou 2, ladite composition comprenant en outre des acides gras (monoinsaturés et polyinsaturés, en particulier acides gras oméga-3) ou des glucides.

4. Procédé selon les revendications 1 ou 2, ladite composition comprenant en outre un antioxydant choisi parmi acide lipoïque, cystéine, cystine, méthionine, S-adénosyl-méthionine, taurine, glutathion ou sources naturelles de ceux-ci, ou caroténoïdes, ubiquinones, catéchines de thé, extraits de café contenant polyphénols et/ou diterpènes, extraits de pépin de raisin riches en proanthocyanidines, extraits d'épice, extraits de soja et acide ursodésoxycholique, acide ursolique, ginseng et ginsenosides et sources naturelles de ceux-ci.

5. Procédé selon l'une des revendications 1 à 4, qui comprend de la carnitine.

6. Procédé selon l'une des revendications 1-5, qui comprend de l'extrait de gingko.
